(19) Europäisches Patentamt
European Patent Office
Office européen des brevets

(11) Veröffentlichungsnummer : **0 219 082 B1**

(12) # EUROPÄISCHE PATENTSCHRIFT

(45) Veröffentlichungstag der Patentschrift : **03.01.90**

(21) Anmeldenummer : **86114105.9**

(22) Anmeldetag : **11.10.86**

(51) Int. Cl.[5] : **C 01 B 6/24**, C 07 B 41/02, C 07 B 31/00, C 07 C 1/22, C 07 C 29/14, C 07 C 29/136, C 07 C 29/132

(54) **Verfahren zur Herstellung von Halogen-Magnesium-Alanat und dessen Verwendung.**

(30) Priorität : **16.10.85 DE 3536797**

(43) Veröffentlichungstag der Anmeldung : **22.04.87 Patentblatt 87/17**

(45) Bekanntmachung des Hinweises auf die Patenterteilung : **03.01.90 Patentblatt 90/01**

(84) Benannte Vertragsstaaten : **AT BE CH DE ES FR GB IT LI LU NL**

(56) Entgegenhaltungen :
**EP-A- 0 003 564**
**INORGANIC CHEMISTRY, MAGNESIUM ALUMINUM HYDRIDE, Band 9, Nr. 2, Februar 1970, Seiten 325-332, American Chemical Society, Easton, PA 18042, US; E.C. ASHBY et al.: "Concerning the preparation of magnesium aluminum hydride. A study of the reactions of lithium and sodium aluminum hydrides with magnesium halides in ether solvents"**

(73) Patentinhaber : **Studiengesellschaft Kohle mbH
Kaiser-Wilhelm-Platz 1
D-4330 Mülheim/Ruhr (DE)**

(72) Erfinder : **Bogdanovic, Borislav, Dr.
Kaiser-Wilhelm-Platz 1
D-4330 Mülheim/Ruhr (DE)**
Erfinder : **Schwickardi, Manfred
Kaiser-Wilhelm-Platz 1
D-4330 Mülheim/Ruhr (DE)**

(74) Vertreter : **von Kreisler, Alek, Dipl.-Chem. et al
Deichmannhaus am Hauptbahnhof
D-5000 Köln 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 219 082 B1

**Beschreibung**

Die Erfindung betrifft ein Verfahren zur einfachen Herstellung von Halogen-Magnesium-Alanat $XMgAlH_4$ (X = Cl, Br, I) und dessen Verwendung.

Reduktionen mit komplexen Metallhydriden sind Standardreaktionen der organischen Chemie (vgl. z.B. N.G. Gaylord, « Reduction with Complex Metal Hydrides », Interscience Publishers, Inc., New York 1956). Das etherlösliche $LiAlH_4$ wird dabei am häufigsten verwendet. Prinzipiell sollte die Substitution des teuren Lithiums durch das billigere Magnesium zu einer Kostenreduzierung führen.

Es hat deshalb nicht an Versuchen gefehlt, $Mg(AlH_4)_2$ zu synthetisieren, in der Literatur findet man widersprüchliche Angaben zu seinen Eigenschaften. Ein in Ether lösliches $Mg(AlH_4)_2$ wurde erstmals von Wiberg und Bauer (Z. Naturforsch. 5B, 397 (1950), Z. Naturforsch. 7B, 131 (1952), DE-845 338) beschrieben :

$$2\,LiAlH_4 + MgBr_2 \longrightarrow Mg(AlH_4)_2 + 2\,LiBr$$

$$4\,MgH_2 + 2\,AlCl_3 \longrightarrow Mg(AlH_4)_2 + 3\,MgCl_2$$

$$MgH_2 + 2\,AlH_3 \longrightarrow Mg(AlH_4)_2$$

Das $MgH_2$ für diese Umsetzungen wurde durch Thermolyse von Grignard- bzw. Diorganomagnesiumverbindungen gewonnen. Dabei fallen neben Magnesiumhalogenid noch Alkene als unerwünschte Nebenprodukte an. Eine Synthese ausgehend von dem teuren Lithiumalanat ist jedoch unerwünscht. Nach A. Hertwig (DE -921 986 (1954) ; C. A. 52, 11371 d)) führt die Umsetzung von Grignardverbindungen mit Wasserstoff zu Chlor-Magnesium-Alanat, $ClMgAlH_4$, das durch Thermolyse in Magnesiumalanat überführt werden kann.

$$4\,RMgX + AlX_3 + 4\,H_2 \longrightarrow XMg(AlH_4) + 3\,MgX_2 + 4\,RH$$

$$2\,XMg(AlH_4) \longrightarrow Mg(AlH_4)_2 + MgX_2$$

Die Umsetzung einer Mischung von $MgH_2$ und Aluminium mit Aluminiumchlorid führt zu $Mg(AlH_4)_2$ (GB-785 348). Die Umsetzung von $MgH_2$ mit Aluminium und Wasserstoff unter drastischen Reaktionsbedingungen (J. C. Snyder, U. S. Pat. 3 387 948 (1962) liefert $Mg(AlH_4)_2$ in unbefriedigenden Ausbeuten. Alle beschriebenen Verfahren genügen technischen Ansprüchen nicht.

Das später (J. Plesek, S. Hermanek, Coll, Czech. Chem. Comm. 31, 3060 (1966) aus Magnesiumhalogenid und Natriumalanat synthetisierte $Mg(AlH_4)_2$ besaß im Gegensatz zu früheren Beschreibungen eine äußerst schlechte Löslichkeit in Ethern. Nach Ashby et al. (Inorg. Chem. 9, 325 (1970)) entsteht bei den Umsetzungen von Lithiumalanat mit Magnesiumbromid bzw. Magnesiumhydrid mit Aluminiumchlorid als Produkt ein lösliches Halogen-Magnesium-Alanat, $XMg(AlH_4)$, das auch aus Hydridmagnesiumchlorid und Alan zugänglich ist (Inorg. Chem. 16, 2941 (1977)). Die wahre Konstitution des Reaktionsproduktes wurde also von Wiberg, Hertwig und Snyder nicht erkannt.

Die Substitution des teuren Lithiumalanates gegen das billigere, lösliche Halogen-Magnesium-Alanat erscheint immer noch wünschenswert. Die Realisierung dieses Konzepts scheiterte, da bisher eine effiziente Synthese für das billige Ausgangsprodukt Magnesiumhydrid fehlte.

Nach der EP 0 003 564 läßt sich metallisches Magnesium mit Hilfe von homogenen Übergangsmetallkatalysatoren unter milden Bedingungen zu Magnesiumhydrid hydrieren, das im Gegensatz zu Magnesiumhydrid, hergestellt nach dem herkömmlichen Verfahren (Hochtemperaturhydrierung), eine hohe Reaktivität aufweist. Dieses Verfahren zur Herstellung von Hydriden des Magnesiums mit Hilfe von Wasserstoff und Übergangsmetallen als Katalysatoren ist dadurch gekennzeichnet, daß man Magnesium in Gegenwart eines Katalysators, bestehend aus einem Halogenid eines Metalls der IV. bis VIII. Nebengruppe des periodischen Systems und einer magnesiumorganischen Verbindung bzw. eines Magnesiumhydrids sowie gegebenenfalls in Gegenwart eines polycyclischen Aromaten oder eines teritären Amins sowie gegebenenfalls in Gegenwart eines Magnesiumhalogenids $MgX_2$ mit X = Cl, Br, I, mit Wasserstoff umsetzt.

In den Unteransprüchen der EP 0 003 564 werden bevorzugte Ausführungsformen beansprucht, nämlich daß die Umsetzung in Gegenwart eines Lösungsmittels, bevorzugt in Tetrahydrofuran, durchgeführt wird, daß bei Drucken von 1 bis 300 bar und bei Temperaturen von 0 bis 200 °C gearbeitet wird, daß das Verhältnis Mg : Übergangsmetall wie $10^4$ bis 10 : 1 und das Verhältnis Übergangsmetall : magnesiumorganischer Verbindung bzw. Magnesiumhydrid wie 0,1 : 1 bis 10 : 1 gewählt wird, als Übergangsmetallhalogenide Chrom-, Titan- bzw. Eisenhalogenide eingesetzt werden, als magnesiumorganische Verbindungen Magnesiumanthracen verwendet wird, als polycyclische Aromaten Anthracen, Tetracen bzw. Benzanthracen und als tertiäre Amine $NR_3$ solche mit R = Alkyl oder Cycloalkylgruppen eingesetzt werden und daß gegebenenfalls bei der Umsetzung des Magnesiums dessen Halogenide $MgX_2$ mit X = Cl, Br, I, im Verhältnis Mg : $MgX_2$ = 1 : 1 zugesetzt werden.

Es wurde nun gefunden, daß sich das nach diesem Verfahren leicht zugängliche hochaktive Magnesiumhydrid in hervorragender Weise zur Erzeugung von Halogen-Magnesium-Alanaten aus Aluminiumhalogeniden eignet.

Dementsprechend betrifft die vorliegende Erfindung ein Verfahren zur Herstellung von Halogen-Magnesium-Alanaten $XMgAlH_4$ (X = Cl, Br, I) aus Aluminiumhalogenid und Magnesiumhydrid, dadurch gekennzeichnet, daß Aluminiumhalogenid $AlX_3$ (X = Cl, Br, I) mit einem Magnesiumhydrid in einem Lösungsmittel umgesetzt wird, wobei dieses Magnesiumhydrid hergestellt ist aus Magnesium und Wasserstoff in einem Lösungsmittel in Gegenwart homogener Katalysatoren, bestehend aus einem Halogenid eines Metalls der IV. bis VIII. Nebengruppe des periodischen Systems und einer magnesiumorganischen Verbindung bzw. eines Magnesiumhydrids sowie in Gegenwart eines polycyclischen Aromaten oder eines tertiären Amins sowie gegebenenfalls in Anwesenheit eines Magnesiumhalogenids $MgX_2$ (X = Cl, Br, I).

Die Reaktion verläuft in aprotischen, organischen Lösungsmitteln, bevorzugt cyclischen oder linearen Ethern bzw. Polyethern wie Tetrahydrofuran (THF) oder Glyme bereits unterhalb 0 °C und liefert Halogen-Magnesium-Alanate in hohen Ausbeuten. Besonders bevorzugt wird THF.

Die Umsetzung des Aluminiumhalogenids mit dem Magnesiumhydrid wird vorzugsweise im Temperaturbereich von — 80 °C bis 150 °C, besonders bevorzugt von — 10 °C bis 60 °C durchgeführt.

Die Kombination der beiden Verfahren ermöglicht eine Zweistufensynthese von Halogen-Magnesium-Alanaten aus den billigen Rohstoffen Magnesium, Wasserstoff und Aluminiumhalogenid, wie nachstehend beispielhaft erläutert :

$$Mg + H_2 \xrightarrow{\text{THF, Kat.}} MgH_2$$

Kat. : 1 mol-% Anthracen, 1 mol-% $CrCl_3$ oder $TiCl_4$

$$2\,MgH_2 + AlX_3 \xrightarrow{\text{THF}} XMg(AlH_4) + MgX_2$$

X =Cl, Br, I

Alternativ besteht die Möglichkeit, das nach der EP 0 003 564 hergestellte $MgH_2$ in fester Form zu isolieren und anschließend in einem anderen geeigneten Lösungsmittel mit Aluminiumhalogeniden zur Reaktion einzusetzen.

Die Herstellung von $ClMg(AlH_4)$ gelingt nach eigenen Untersuchungen nicht mit handelsüblichem $MgH_2$, das aus den Elementen bei hohen Temperaturen hergestellt wurde. Ein derartiges Magnesiumhydrid liefert auch bei Aktivierung durch Mahlen in einer Glaskugelmühle bei der Reaktion mit ungesättigten Substraten keine Reduktionsprodukte, wie aus dem Vergleichsbeispiel hervorgeht.

Das gemäß der vorliegenden Erfindung hergestellte Chlor-Magnesium-Alanat kann direkt als Lösung oder nach Entfernen des Lösungsmittels in fester Form zu Reduktionszwecken verwendet werden. Das als Nebenprodukt anfallende $MgCl_2$ stört bei den Folgereaktionen nicht, im Falle des isolierten Materials wird die Pyrophorizität des Materials noch gesenkt. Wird die Abtrennung des gebildeten $MgCl_2$ jedoch gewünscht, so ist dies durch Zusatz von Dioxan möglich.

Das aus $MgH_2$ und $AlCl_3$ leicht zugängliche $ClMg(AlH_4)$ ist ein sehr gutes Reduktionsmittel für funktionelle organische Gruppen. Es zeigte sich (vgl. Beispiele), daß Aldehyde, Ester, Ketone, Alkylhalogenide, Carbonsäuren und -anhydride in sehr guten Ausbeuten reduziert werden. Ein Wechsel des Lösungsmittels für die Reduktionen ist möglich.

Die erzielten Ausbeuten sind oft besser, als die von Wiberg und Bauer (Chem. Ber. 85, 593 (1952)) erzielten. Das Reduktionsvermögen von $ClMg(AlH_4)$ ist mit dem des $LiAlH_4$ vergleichbar, der Ersatz des teuren Lithiums durch das billige Magnesium möglich und wünschenswert. Ein weiterer Vorteil dieser Methode ist, daß die Hydrierung von Magnesium und anschließende Reduktion von funktionellen organischen Verbindungen in einem Eintopfverfahren in z. B. THF als Reaktionsmedium durchgeführt werden kann.

Die Erfindung wird durch die nachstehenden Beispiele näher erläutert, ohne auf sie beschränkt zu sein. Alle Versuche wurden in absoluten Lösungsmitteln unter Argon als Schutzgas durchgeführt.

## Beispiel 1

48 ml einer 4,12 molaren $MgH_2$-Suspension (hergestellt nach EP 0 003 564 mit Chromkatalysator ; Mg : Anthracen : $CrCl_3$ = 100 : 1 : 1) wurden in einem 250 ml-Dreihalskolben, der mit Tropftrichter, Rückflußkühler und Innenthermometer versehen ist, vorgelegt. Zu der gut gerührten Suspension wurden innerhalb 30 min. 8,85 g (66 mmol) $AlCl_3$, gelöst in 60 ml THF, so zugetropft, daß die Innentemperatur nicht über 30 °C ansteigt (äußere Kühlung). Dann wurden sofort 25,3 ml (239 mmol) 3-Pentanon in 20 min. zugetropft (exotherme Reaktion, äußere Kühlung). Man ließ 1 h bei Raumtemperatur nachreagieren, dann wurden die flüchtigen Reaktionsprodukte im Ölpumpenvakuum entfernt. Der Rückstand wurde in 60 ml Toluol suspendiert und mit $H_2O$ und HCl zersetzt. Die organische Phase wurde abgetrennt, die wässrige zweimal mit Toluol extrahiert. Die vereinigten organischen Phasen wurden bei 20 bis 120 °C/0,1 mbar destilliert.

Auswaage : 172,77 g

Ein aliquoter Teil wurde mit n-Octan versetzt und gaschromatographisch untersucht.

Ausbeute : 19,98 g 3-Pentanol (95 % d. Th.)

Beispiel 2

Der Versuch wurde wie in Beispiel 1 mit 60 ml einer 3,42 molaren $MgH_2$-Suspension, 6,63 g $AlCl_3$ (50 mmol) in 59 ml THF und 17,2 ml (169 mmol) Benzaldehyd durchgeführt.
Ausbeute : 16,96 g (157 mmol) Benzylalkohol ; 93 % d. Th.

Beispiel 3

Der Versuch wurde wie in Beispiel 1 mit 50 ml einer 4,12 molaren $MgH_2$-Suspension, 8,02 g $AlCl_3$ (60 mmol) in 60 ml THF und 15,5 ml (108 mmol) Benzoesäureethylester durchgeführt.
Ausbeute : 11,67 g (108 mmol) Benzylalkohol ; 100 % d. Th.

Beispiel 4

Der Versuch wurde wie in Beispiel 1 mit 70 ml einer 2,94 molaren $MgH_2$-Suspension, 8,5 g $AlCl_3$ (64 mmol) in 60 ml THF und 34 ml (216 mmol) 1-Bromheptan durchgeführt.
Ausbeute : 19,34 g (193 mmol) n-Heptan ; 89 % d. Th.

Beispiel 5

Der Versuch wurde wie in Beispiel 1 mit 30 ml einer 6,33 molaren $MgH_2$-Suspension, 4,08 g $AlCl_3$ (30,6 mmol) in 48 ml THF und 8,5 ml (104 mmol) Crotonaldehyd durchgeführt.
Ausbeute : 6,79 g (94,2 mmol) Crotylalkohol ; 91 % d. Th.

Beispiel 6

Der Versuch wurde wie in Beispiel 1 mit 70 ml einer 3,01 molaren $MgH_2$-Suspension, 9,28 g $AlCl_3$ (70 mmol) in 74 ml THF und 9,4 ml (59,3 mmol) Octansäure durchgeführt.
Ausbeute : 7,15 g (54,9 mmol) 1-Octanol ; 93 % d. Th.

Beispiel 7

Der Versuch wurde wie in Beispiel 1 mit 42 ml einer 5,88 molaren $MgH_2$-Suspension, 11,0 g $AlCl_3$ (82,6 mmol) in 100 ml THF und 6,0 ml (46,8 mmol) Propionsäureanhydrid durchgeführt.
Ausbeute : 5,371 g (89,4 mmol) 1-Propanol ; 95 % d. Th.

Beispiel 8

Der Versuch wurde wie in Beispiel 1 mit 40 ml einer 2,7 molaren $MgH_2$-Suspension, 4,77 g $AlCl_3$ (36 mmol) in 44 ml THF und 10,4 ml (102 mmol) Benzaldehyd bei 65 °C durchgeführt.
Ausbeute : 8,87 g (82,0 mmol) Benzylalkohol ; 80 % d. Th.

Beispiel 9

Der Versuch wurde wie in Beispiel 1 mit 2,40 g (91,1 mmol) isoliertem und getrocknetem $MgH_2$, 3,92 g $AlCl_3$ (29,4 mmol) in 66 ml THF und 10,1 ml (100 mmol) Benzaldehyd durchgeführt.
Ausbeute : 8,61 g (79,6 mmol) Benzylalkohol ; 80 % d. Th.

Beispiel 10

Der Versuch wurde wie in Beispiel 1 mit 2,40 g (91,1 mmol) isoliertem und getrocknetem $MgH_2$, das mit Titankatalysator hergestellt wurde, 3,92 g $AlCl_3$ (29,4 mmol) in 66 ml THF und 10,1 ml (100 mmol) Benzaldehyd durchgeführt.
Ausbeute : 8,61 g (79,6 mmol) Benzylalkohol ; 80 % d. Th.

Vergleichsbeispiel

Der Versuch wurde mit 6,11 g (197 mmol) 85 %igem, käuflichem $MgH_2$, das 2 h durch Rühren in einer Glaskugelmühle aktiviert wurde, 6,74 g $AlCl_3$ (50,5 mmol) in 60 ml THF und 13,0 ml (90,5 mmol) Benzoesäureethylester wie in Beispiel 1 durchgeführt. Neben Ausgangsverbindung ließ sich gaschromatographisch kein Benzylalkohol nachweisen.

**Patentansprüche**

1. Verfahren zur Herstellung von Halogen-Magnesium-Alanaten $XMgAlH_4$ (X = Cl, Br, I) aus Aluminiumhalogenid und Magnesiumhydrid, dadurch gekennzeichnet, daß Aluminiumhalogenid $AlX_3$ (X = Cl, Br, I) mit einem Magnesiumhydrid in einem Lösungsmittel umgesetzt wird, wobei dieses Magnesiumhydrid hergestellt ist aus Magnesium und Wasserstoff in einem Lösungsmittel in Gegenwart homogener Katalysatoren, bestehend aus einem Halogenid eines Metalls der IV. bis VIII. Nebengruppe des periodischen Systems und einer magnesiumorganischen Verbindung bzw. eines Magnesiumhydrids sowie in Gegenwart eines polycyclischen Aromaten oder eines tertiären Amins sowie gegebenenfalls in Anwesenheit eines Magnesiumhalogenids $MgX_2$ (X = Cl, Br, I).

2. Verfahren nach Anspruch 1, dadurch gekennzeichnet, daß als Lösungsmittel aprotische, organische Lösungsmittel, bevorzugt offenkettige und cyclische Mono- und Polyether wie THF oder Glykoldimethylether, und insbesondere THF verwendet werden.

3. Verfahren nach Ansprüchen 1 und 2, dadurch gekennzeichnet, daß die Umsetzung des Aluminiumhalogenids mit dem Magnesiumhydrid im Temperaturebereich von — 80 °C bis 150 °C, bevorzugt von — 10 °C bis 60 °C durchgeführt wird.

4. Verfahren nach Ansprüchen 1 bis 3, dadurch gekennzeichnet, daß man zweistufig arbeitet, wobei man in einer ersten Stufe das Magnesiumhydrid herstellt und dann in einer zweiten Stufe im gleichen Lösungsmittel, vorzugsweise THF, mit dem Aluminiumhalogenid umsetzt.

5. Verwendung des nach Ansprüchen 1 bis 4 gebildeten Halogen-Magnesium-Alanats, direkt oder nach Isolierung im gewünschten Lösungsmittel, zur Reduktion funktioneller organischer Verbindungen.

6. Verwendung nach Anspruch 5, dadurch gekennzeichnet, daß die Bildung von Halogen-Magnesium-Alanat und die anschließende Reduktion funktioneller organischer Verbindungen als Eintopfreaktion durchgeführt wird.

## Claims

1. A process for the preparation of halogen magnesium alanates $XMgAlH_4$ (X = Cl, Br, I) from aluminium halide and magnesium hydride, characterised in that aluminium halide $AlX_3$ (X = Cl, Br, I) is reacted with a magnesium hydride in a solvent, which magnesium hydride has been prepared from magnesium and hydrogen in a solvent in the presence of homogeneous catalysts consisting of a halide of a metal of subgroups IV to VIII of the periodic system and an organomagnesium compound or a magnesium hydride and in the presence of a polycyclic aromatic compound or a tertiary amine and optionally in the presence of a magnesium halide $MgX_2$ (X = Cl, Br, I).

2. Process according to Claim 1, characterised in that the solvents used are aprotic, organic solvents, preferably open chain and cyclic mono and polyethers such as THF or glycol dimethyl ether and more particularly THF.

3. Process according to Claims 1 and 2, characterised in that the reaction of the aluminium halide with the magnesium hydride is carried out in the temperature range of from — 80 °C to 150 °C, preferably from — 10 °C to 60 °C.

4. Process according to Claims 1 to 3, characterised in that it is carried out in two steps, the magnesium hydride being prepared in a first step and then reacted with the aluminium halide in a second step in the same solvent, preferably in THF.

5. Use of the halogen magnesium alanate formed according to Claims 1 to 4 for the reduction of functional organic compounds, either directly or after isolation in the desired solvent.

6. Use according to Claim 5, characterised in that the formation of halogen magnesium alanate and the subsequent reduction of functional organic compounds are carried out as a single vessel reaction.

## Revendications

1. Procédé de préparation d'alanates d'halogénomagnésium $XMgAlH_4$ (X représentant Cl, Br ou I) à partir d'un halogénure de magnésium et d'un hydrure de magnésium, caractérisé en ce qu'on fait réagir un halogénure d'aluminium $AlX_3$ (X représentant Cl, Br ou I) avec un hydrure de magnésium dans un solvant, cet hydrure de magnésium ayant été préparé à partir de magnésium et d'hydrogène dans un solvant en présence de catalyseurs homogènes, consistant en un halogénure d'un métal du sous-groupe IV à VIII du Tableau Périodique et d'un composé organique du magnésium ou d'un hydrure de magnésium ainsi qu'en présence d'un hydrocarbure aromatique polycyclique ou d'une amine tertiaire ainsi que, éventuellement, en présence d'un halogénure de magnésium $MgX_2$ (X représentant Cl, Br ou I).

2. Procédé selon la revendication 1, caractérisé en ce qu'on utilise comme solvant des solvants organiques aprotiques, de préférence des monoéthers ou des polyéthers à chaînes droites ou cycliques, comme THF (tétrahydrofuranne) ou de l'éther diméthylique de glycol (glyme) et en particulier THF.

3. Procédé selon les revendications 1 et 2, caractérisé en ce qu'on conduit la réaction de l'halogénure d'aluminium avec l'hydrure de magnésium dans un intervalle de températures allant de — 80 °C à + 150 °C, de préférence de — 10 °C à + 60 °C.

4. Procédé selon les revendications 1 à 3, caractérisé en ce qu'on travaille en deux étapes, en

préparant dans une première étape l'hydrure de magnésium et en le faisant ensuite réagir dans une seconde étape dans le même solvant, avantageusement THF, avec l'halogénure d'aluminium.

5. Utilisation de l'alanate d'halogéno-magnésium, formé selon les revendications 1 à 4, directement ou après isolement dans des solvants voulus, pour réduire des composés organiques comportant des groupes fonctionnels.

6. Utilisation selon la revendication 5, caractérisée en ce qu'on conduit la formation de l'alanate d'halogéno-magnésium et la réduction subséquente de composés organiques comportant des groupes fonctionnels sous forme de réactions effectuées dans un seul et même récipient.